# EUROPEAN PATENT SPECIFICATION

(11) **EP 1 935 898 B1**
(45) Date of publication and mention of the grant of the patent: **19.03.2014**
(21) Application number: 07024615.2
(22) Date of filing: 19.12.2007
(51) Int. Cl.: C07J 41/00

(54) **Process for the preparation of 17alpha-cyanomethyl-17beta-hydroxy steroids**
Verfahren zur Herstellung von 17alpha-Cyanomethyl-17beta-Hydroxysteroiden
Procédé pour la préparation de 17-alpha-cyanométhyl-17-béta-hydroxy stéroïdes

(30) Priority: 22.12.2006 IT MI20062504
(43) Date of publication of application: 25.06.2008
(73) Proprietor: OLON S.p.A., 20090 Rodano (MI) (IT)
(72) Inventor: Alpegiani, Marco, 20132 Milano (IT); Cabri, Walter, 20089 Rozzano (MI) (IT); Cristiano, Tania, 20162 Milano (IT); Strigaro, Roberto, 20162 Milano (IT); Danelli, Tamara Ines, 24058 Romano di Lombardia (Bergamo) (IT)
(74) Representative: Minoja, Fabrizio

(56) References cited:
- WO-A1-01/78606
- US-A- 5 438 134
- D. H. R. BARTON ET AL: "On the stability and radical deoxygenation of tertiary xanthates", TETRAHEDRON LETTERS., vol. 34, no. 17, 1993, pages 2733-2736, XP002681194, NLELSEVIER SCIENCE PUBLISHERS, AMSTERDAM. ISSN: 0040-4039
- IMAMOTO T ET AL: "Organocerium reagents. Nucleophilic addition to easily enolizable ketones", TETRAHEDRON LETTERS, ELSEVIER, AMSTERDAM, NL, vol. 25, no. 38, 1 January 1984 (1984-01-01), pages 4233-4236, XP026636982, ISSN: 0040-4039, DOI: 10.1016/S0040-4039(01)81404-0 [retrieved on 1984-01-01]
- HSING-JANG LIU ET AL: "FORMATION AND EFFICIENT ADDITION OF CERIUM(III) DERIVATIVE OF ACETONITRILE TO KETONES", TETRAHEDRON LETTERS, ELSEVIER, AMSTERDAM, NL, vol. 32, no. 40, 30 September 1991 (1991-09-30), pages 5473-5476, XP000226489, ISSN: 0040-4039, DOI: 10.1016/0040-4039(91)80061-A

## Description

### Field of the invention

The present invention relates to a process for the preparation of 17α-cyanomethyl-17β-hydroxy steroids, in particular to a process for the preparation of dienogest (I)

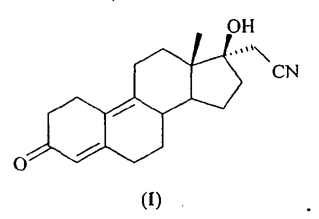

### Background of the invention

Dienogest (I) is a synthetic steroid of the nor-series used in the treatment endometriosis. From the structural point of view, it is characterized by the presence of a hydroxy group and a cyanomethyl group at the alpha and beta position of the D ring.

The introduction of the cyanomethyl group into steroidal derivatives can be carried out by opening of a 17-spiro oxirane derivative with cyanides, according to the following reaction scheme: 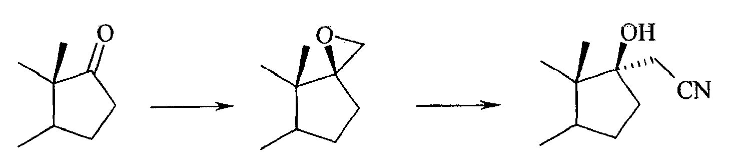 as described, for example, in EP776904 and DD 160418.

US 5438134 teaches the introduction of the cyanomethyl group into 17-keto steroids by reaction with lithium acetonitrile.

### Disclosure of the Invention

It has now been found that 17α-cyanomethyl-17β hydroxy steroids, in particular dienogest (I) 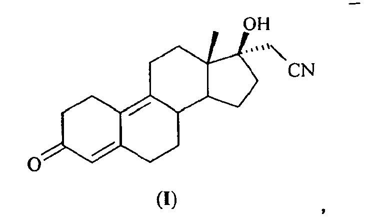 can be conveniently prepared by reacting 17-ketosteroids with cyanomethyl cerium derivatives of formula CNCH₂CeX₂, in which X is a halide selected from fluorine, chlorine, bromine or iodine, preferably chlorine, or an alkoxide, preferably an ethoxy, isopropoxy or *tert*-butoxy group.

The preferred reagent is CNCH₂CeCl₂, which can be prepared *in* situ by reaction between acetonitrile and an alkyl-lithium or alkyl-magnesium compound, which is added with CeCl₃, according to what disclosed for example by Hsing-Jang in Tetrahedron Letters 1991, 5473-5476. This reaction can also give rise to aggregates of the cerium salt with LiCl and MgCl₂, which are not recovered and which react in the same way as CNCH₂CeCl₂.

The reaction is usually carried out with 1 to 10 equivalents of cerium salt, preferably from 2 to 5 equivalents, in an anhydrous organic solvent, preferably an ether, such as tetrahydrofuran, 2-methyl tetrahydrofuran, dioxane, 1,2 dimethoxyethane (diglyme), methylal, ethylal, methyl *tert*-butyl ether, *n*-butyl ether, at temperatures ranging from -78 to 40°C, or a hydrocarbon, such as toluene or xylene.

In the case of dienogest, the keto group at the 3-position needs to be suitably protected, for example as ketal or enolether, which is hydrolysed after the reaction with the cerium salt.

With respect to lithium acetonitrile, the cerium salts used in the process of the invention have the advantage of being stable at temperatures above -30°C, therefore they are more suitable for industrial scale production. Moreover, it has been observed that, with respect to the preparation of dienogest carried out under the conditions described in US 5,438,134, i.e. using lithium acetonitrile, the process of the invention allows to obtain higher yields and purity.

The invention is herein after illustrated in greater detail by means of some examples.

### EXAMPLES

### Example 1

A 2.5 N solution of 21 ml *n*-butyllithium in 50 ml tetrahydrofuran is dropped at 0°C into a solution of 10 ml hexamethyldisilazane in 50 ml tetrahydrofuran, under nitrogen atmosphere. After 30 minutes a solution of 2.7 ml acetonitrile in 50 ml tetrahydrofuran is added at -78°C and stirring is continued for 20 minutes, Thereafter, 12.8 g anhydrous cerium trichloride is loaded, stirring is continued for 30 minutes and finally a solution of 5 g 3,3-dimethoxyestra-5(10),9(11)-dien-17-one in 25 ml tetrahydrofuran is added, allowing the temperature to raise to 0°C in 1 hour. The reaction is quenched by addition of 14.6 ml acetic acid and 158 ml water and the phases are separated. The organic phase is added with 16.2 ml hydrochloric acid and allowed to react at 40°C for 2 hours. The phases are separated and the organic one is reduced to a residue under vacuum. The resulting crude product is purified by flash chromatography. 4 g dienogest is obtained.

### Example 2

A 2.5 N solution of 22 ml n-butyllithium in 50 ml tetrahydrofuran is dropped at 0°C into a solution of 10.5 ml hexamethyldisilazane in 50 ml tetrahydrofuran, under nitrogen atmosphere. After 30 minutes a solution of 2.9 ml acetonitrile in 50 ml tetrahydrofuran is added at -78°C and left under stirring for 20 minutes. 13.6 g anhydrous cerium trichloride is then loaded and stirring is continued for 30 minutes. Finally, a solution of 5 g 3-ethoxy-estra-3,5(10),9(11)-trien-17-one in 25 ml tetrahydrofuran is added and the temperature is allowed to raise to 0°C in 1 hour The reaction is quenched by addition of 15.5 ml acetic acid and 167.5 ml water and the phases are separated. The organic phase is added with 17.2 ml of hydrochloric acid and allowed to react at 40°C for 2 hours. The phases are separated and the organic one is reduced to residue under vacuum. The resulting crude product is purified by flash chromatography. 3.8 g dienogest is obtained.

### Example 3

13 ml hexamethyldisilazane is dropped into a solution of 100 ml tetrahydrofuran and 26.4 ml 2.5 N n-butyllithium at 0°C, under nitrogen atmosphere. After 30 minutes, 3.4 ml acetonitrile is added at -78°C and stirring is continued for 30 minutes. Thereafter, 17.76 g anhydrous cerium trichloride is loaded and stirring is continued at -78°C for 30 minutes. Finally, a solution of 10 g 3,3-dimethoxyestra-5(10),9(11)-dien-17-one in 50 ml tetrahydrofuran is added and stirring is continued at -78°C for 30 minutes. The temperature is then allowed to raise to 0°C in 2 hours. The reaction is quenched by addition of a solution of 10.3 ml acetic acid in 108 ml water and the phases are separated. The organic phase is added with 62.8 ml 10% hydrochloric acid and the reaction is continued at 60°C for 90 minutes. The reaction is quenched by addition of 170 ml of an aqueous saturated solution of sodium bicarbonate and the phases are separated. The aqueous phase is extracted with 2-methyl tetrahydrofuran. The organic phase is reduced under vacuum and the residue is crystallized from acetone. The resulting crude product is purified by flash chromatography. 5 g dienogest is obtained.

### Example 4

13.8 ml hexamethyldisilazane is dropped into a solution of 100 ml tetrahydrofuran and 28 ml 2.5 N n-butyllithium at 0°C, under nitrogen atmosphere. After 30 minutes 3.68 ml acetonitrile is added at -78°C and stirring is continued for 30 minutes 18.84 g anhydrous cerium trichloride is then added and stirring is continued at -78°C for 30 minutes. Finally, a solution of 10 g 3-ethoxy-estra-3,5(10),9(11)-trien-17-one in 50 ml tetrahydrofuran is added and stirring is continued at -78°C for 30 minutes. The temperature is then allowed to raise gradually to 0°C in 2 hours. The reaction is quenched by addition of a solution of 10.92 ml acetic acid in 114.8 ml water and the phases are separated. The organic phase is added with 66.6 ml 10% hydrochloric acid and allowed to react at 60°C for 90 minutes. The reaction is quenched by addition of 174 ml of an aqueous saturated solution of sodium bicarbonate and the phases are separated. The aqueous phase is extracted with 2-methyl tetrahydrofuran. The organic phase is evaporated under vacuum and the residue is crystallized from acetone. The resulting crude product is purified by flash chromatography. 4.6 g dienogest is obtained.

## Claims

1. Process for the preparation of 17α-cyanomethyl-17β hydroxy steroids comprising the reaction of 17-keto steroids with cyanomethyl cerium derivatives of formula CNCH₂CeX₂, in which X is a halide selected from fluorine, chlorine, bromine and iodine, or an alkoxide.

2. The process according to claim 1 wherein X is chlorine.

3. The process according to claim 1 wherein X is an ethoxy, isopropoxy or *tert-*butoxy group.

4. The process as claimed in claim 1 wherein the 17α-cyanomethyl-17β hydroxy steroid is dienogest (I):

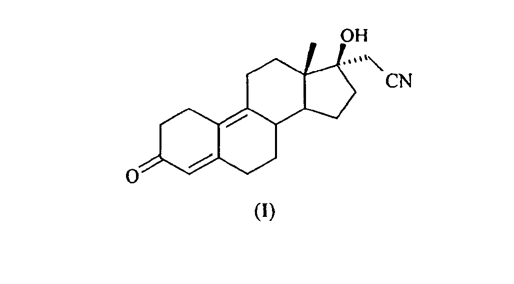

## Patentansprüche

1. Verfahren zur Herstellung von 17α-Cyanomethyl-17β-hydroxy-Steroiden umfassend die Reaktion von 17-Keto-Steroiden mit Cyanomethyl-Cer-Derivaten der Formel CNCH₂CeX₂, worin X ein Halogenid ausgewählt aus Fluor, Chlor, Brom und lod oder ein Alkoxid ist.

2. Verfahren gemäß Anspruch 1, worin X Chlor ist.

3. Verfahren gemäß Anspruch 1, worin X eine Ethoxy-, Isopropoxy- oder tert-Butoxy-Gruppe ist.

4. Verfahren gemäß Anspruch 1, worin das 17α-Cyanomethyl-17β-hydroxy-Steroid Dienogest (I) ist:

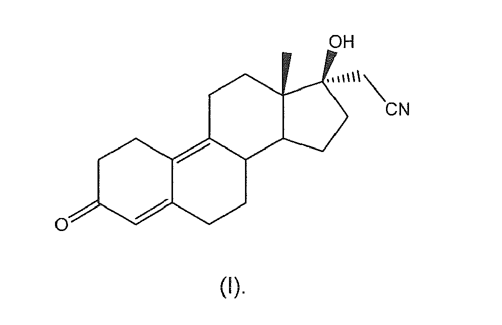

## Revendications

1. Procédé pour la préparation de 17α-cyanométhyl-17β-hydroxystéroïdes comprenant la mise en réaction de 17-cétostéroïdes avec des dérivés de cyanométhylcérium répondant à la formule CNCH₂CeX₂ dans laquelle X représente un halogénure choisi parmi un atome de fluor, un atome de chlore, un atome de brome et un atome d'iode, ou un groupe alcoxyde.

2. Procédé selon la revendication 1, dans lequel X représente un atome de chlore.

3. Procédé selon la revendication 1, dans lequel X représente un groupe éthoxy, un groupe isopropoxy ou un groupe tert-butoxy.

4. Procédé selon la revendication 1, dans lequel le 17α-cyanométhyl-17β-hydroxystéroïde est le diénogest (I) :
